(19) Europäisches Patentamt
European Patent Office
Office européen des brevets

(11) Veröffentlichungsnummer: **0 260 646**
**A2**

(12) **EUROPÄISCHE PATENTANMELDUNG**

(21) Anmeldenummer: 87113437.5

(22) Anmeldetag: 15.09.87

(51) Int. Cl.⁴: C07C 87/50, C07C 87/64, C07C 121/66

(30) Priorität: 17.09.86 DE 3631614

(43) Veröffentlichungstag der Anmeldung: 23.03.88 Patentblatt 88/12

(84) Benannte Vertragsstaaten: BE CH DE ES FR GB IT LI NL

(71) Anmelder: Max-Planck-Gesellschaft zur Förderung der Wissenschaften e.V.
Bunsenstrasse 10
D-3400 Göttingen(DE)

(72) Erfinder: Staab, Heinz A., Prof. Dr.
Schloss-Wolfsbrunnenweg 43
D-6900 Heidelberg(DE)
Erfinder: Elbl, Karin
Neuberg-Strasse 48/3
D-7107 Neckarsulm(DE)

(74) Vertreter: Michaelis, Wolfgang, Dr.
c/o BASF Aktiengesellschaft 38
Carl-Bosch-Strasse
D-6700 Ludwigshafen(DE)

(54) **Tetrakis(dialkylamino)aromaten und deren Charge-Transfer-Komplexe.**

(57) Die neuen Tetrakis(dialkylamino)aromaten der allgemeinen Formeln I bis III

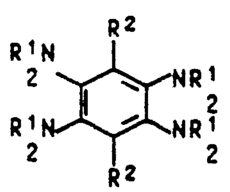

(I)

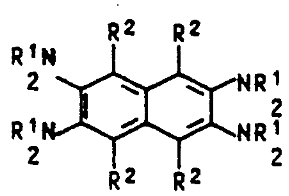

(II)

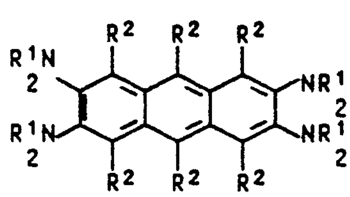

(III)

EP 0 260 646 A2

worin die Reste $R^1$ gleich oder verschieden sein können und für n-$C_1$-$C_6$-Alkyl stehen und die Reste $R^2$ gleich oder verschieden sein können und für Wasserstoff oder $C_1$-$C_6$-Alkyl stehen, sind brauchbar zur Herstellung von Charge-Transfer-Komplexen, welche elektrisch leitfähig sind. Die Verbindungen lassen sich durch Umsetzung der am Stickstoff unsubstituierten Verbindungen mit den entsprechenden Aldehyden in Gegenwart von Natriumcyanoborhydrid herstellen.

## Tetrakis(dialkylamino)aromaten und deren Charge-Transfer-Komplexe

Die Erfindung betrifft Tetrakis(dialkylamino)aromaten, deren Charge-Transfer-Komplexe, ein Verfahren zur Herstellung dieser Verbindungen sowie die Verwendung der Komplexe zur Herstellung leitfähiger Polymerer.

In Ber. Dt. Chem. Ges. 12 (1879) 522, 2071 ist das 1,4-Bis(dimethylamino)benzol, auch als N,N,N'N'-Tetramethyl-para-phenylendiamin bezeichnet, beschrieben. Diese Verbindung ist wegen ihrer großen Donor-Stärke eine der bestuntersuchten Elektronen-Donoren der organischen Chemie.

Das 1,2-Bis(dimethylamino)benzol wurde in Ber. Dt. Chem. Ges. 25 (1892) 2826, beschrieben. Bekannt ist auch das 1,2,4,5-Tetraaminobenzol, vgl. Ber. Dt. Chem. Ges. 20 (1887), 328. Schließlich sind in Angew. Chem. 98 (1986), 460, Untersuchungen über Verbindungen mit räumlich benachbarten Dimethylaminogruppen beschrieben, welche als sogenannte "Protonenschwämme" wirken.

Der Erfindung lag die Aufgabe zugrunde, neue Tetrakis(dialkylamino)aromaten zur Verfügung zu stellen, welche interessante Eigenschaften aufweisen und insbesondere zur Bildung von leitfähigen Charge-Transfer-Komplexen geeignet sind.

Diese Aufgabe wird gelöst durch Bereitstellung von neuen Tetrakis(dialkylamino)aromaten der allgemeinen Formeln Ia bis Ic

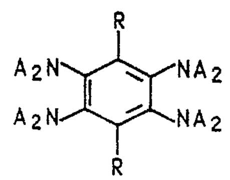

(Ia)

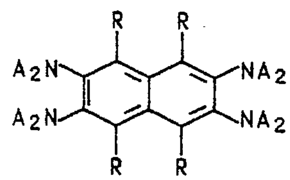

(Ib)

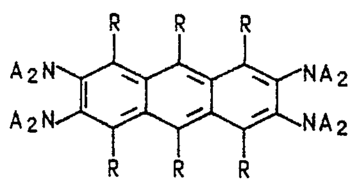

(Ic)

worin die Reste A gleich oder verschieden sein können und für n-$C_1$-$C_6$-Alkyl stehen und die Reste $R^2$ gleich oder verschieden sein können und für Wasserstoff oder $C_1$-$C_6$-Alkyl stehen, und deren Charge-Transfer-Komplexe mit Elektronen-Akzeptoren.

Unter diesen Verbindungen sind die Verbindungen Ia bevorzugt, in denen A für n-$C_1$-$C_4$-Alkyl steht und R Wasserstoff oder $C_1$-$C_4$-Alkyl bedeutet, wobei dem 1,2,4,5-Tetrakis(dimethylamino)benzol die größte Bedeutung zukommt.

Gegenstand der Erfindung ist auch ein Verfahren zur Herstellung der Tetrakis(dialkylamino)aromaten Ia bis Ic und deren Charge-Transfer-Komplexen, das dadurch gekennzeichnet, daß man die entsprechenden N-unsubstituierten Tetramine IIa bis IIc

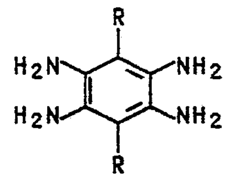

(IIa)

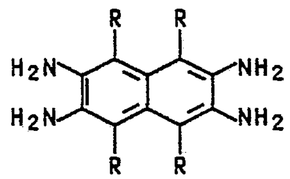

(IIb)

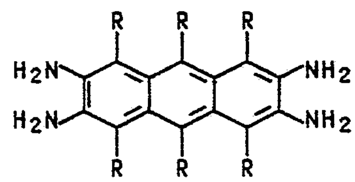

(IIc)

oder deren Säureadditionssalze in einem wasserlöslichen polaren Lösemittel in Gegenwart von Natriumcyanoborhydrid
$NaBH_3CN$
mit einem Aldehyd der allgemeinen Formel III

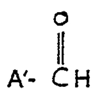

(III)

in der A' für Wasserstoff oder n-$C_1$-$C_5$-Alkyl steht, reagieren läßt und das erhaltene Produkt gegebenenfalls mit einem Elektronen-Akzeptor unter Bildung eines entsprechenden Charge-Transfer-Komplexes umsetzt.

Nach einer bevorzugten Ausführungsform des erfindungsgemäßen Verfahrens, dessen Methodik an sich aus J. Org. Chem. 37 (1972), 1673, bekannt ist, setzt man die Tetramine IIa bis IIc zur Herstellung der N-permethylierten Verbindungen Ia bis Ic mit wäßrigem Formaldehyd um, deren Konzentration zweckmäßigerweise zwischen 30 und 40, besonders zwischen 35 und 38 Gew.%, liegt.

Zur Herstellung der Charge-Transfer-Komplexe der Verbindungen Ia bis Ic kann man den jeweils gewünschten Tetrakis(dialkylamino)aromaten, gelöst oder suspendiert in einem inerten Lösemittel, mit dem entsprechenden Elektronen-Akzeptor in an sich bekannter Weise umsetzen.

Entspricht der Elektronen-Akzeptor einem Anion wie vorzugsweise Halogenid, Trijodid, Nitrat, Perchlorat, Tetrafluorborat, Phosphorhexafluorid, Arsenhexafluorid oder das Anion einer organischen $C_1$-$C_{12}$-Säure, beispielsweise der Essigsäure, Benzoesäure, Terephthalsäure oder p-Toluolsulfonsäure, stellt man die Komplexe zweckmäßigerweise elektrochemisch her, indem man eine organische Lösung des Donators (I) - ein gut geeignetes Lösungsmittel ist häufig Acetonitril - und einem Metall-oder Ammoniumsalz, beispielsweise Silbernitrat, der Elektrolyse unterwirft. Hierbei wird das Kation an der Kathode entladen, währen sich an der Anode der Transfer-Komplex mit bis zu zwei Ladungen pro Molekül I, also beispielsweise $I^{++}$ $(NO_3)_2^-$ bildet.

Eine der möglichen Grenzstrukturen des Donator-Kations läßt sich wie folgt formulieren

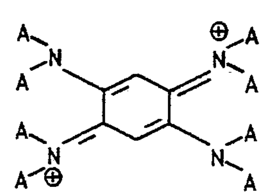

jedoch ist der Sitz der Ladung wegen vollständiger Mesomerie tatsächlich nicht zu lokalisieren.

Ist man erst einmal im Besitz eines dieser Kation-Anion-Komple, kann man das Anion beispielsweise mit Hilfe eines Anionenaustauschers durch ein anderes Anion austauschen.

Unter den Anionen kommt dem Anionenpar Jodid/Trijodid, welches sich durch Umsetzung von (I) mit Iod bildet, besondere Bedeutung zu.

Ursprünglich nicht anionische Akzeptoren, also π-Elektronen-Akzeptoren, - wozu beispielsweise auch Jod zu zählen ist - sind im Hinblick auf die Leitfähigkeitseigenschaften der Komplexe im allgemeinen von größerem Interesse. Solche Verbindungen sind beispielsweise Tetracyano-p-chinodimethan, Tetrachlor-o-benzochinon, Dicyanodichlor-p-benzochinon, Tetracyanoethylen, Nitroarine und Tetracyanobenzol, also allgemein Verbindungen, die aufgrund ihrer Substituenten und Struktureinheiten (Halogen, Nitrogruppen, Cyangruppen, aromatische oder konjugierte Systeme) eine hohe Elektronenaffinität aufweisen.

Die Herstellung dieser Komplexe ist im allgemeinen besonders einfach, da sie sich durch Zusammengeben der Komponenten bilden und dann meistens als Niederschläge nur noch abfiltriert zu werden brauchen.

Da die Leitfähigkeit der Komplexe mit deren Reinheit zunimmt, empfiehlt es sich, die Komponenten, Donor und Akzeptor, in möglichst reiner Form einzusetzen. Als Reinigungsmethoden kommen die Kristallisation und die Destillation in Betracht; das Tetrakis-(dimethylamino)-benzol kann auch durch Sublimation gereinigt werden.

Das Arbeiten unter Schutzgas ist zweckmäßig, da die in Lösung oder Suspension vorliegenden erfindungsgemäßen Verbindungen durch Oxidationsmittel wie Luftsauerstoff leicht unter Grünfärbung oxidiert werden.

Als Lösungsmittel zur Herstellung der erfindungsgemäßen Verbindungen verwendet man polare, mit Wasser mischbare Lösungsmittel, insbesondere Acetonitril. Aber auch andere Lösungsmittel kommen in Betracht, beispielsweise Propionitril, Dimethylsulfoxid und Dimethylformamid.

Die meisten erfindungsgemäßen Charge-Transfer-Komplexe sind wegen ihrer Leitfähigkeit für zahlreiche Anwendungen von großem Interesse. Sie dienen vor allem zur Herstellung leitfähiger Polymerer, zur Herstellung von Batterien auf organischer Basis oder auch zur antistatischen Ausrüstung von Polymeren. Darüber hinaus sind sie wegen ihrer Halbleiter-Eigenschaften in der Elektronik brauchbar.

Als Kunststoffe, in welche die erfindungsgemäßen Charge-Transfer-Komplexe eingearbeitet werden können, kommen die verschiedensten Polymeren in Betracht, so beispielsweise Polyolefine, wie Polyethylen, Homo-und Copolymerisate des Styrols, α-Methylstyrols, p-Methylstyrols, Butadiens, Acryl-und Methacrylnitrils, und von Acrylaten und Methacrylaten, Polyisopren, Polyester, wie Polyethylenterephthalat, Polypyrrole, Polyamide wie Polyamid-6,6, Polycarbonate, Polysulfon, Homo-und Copolymerisate, die Vinylacetat, Vinylcarbazol, Vinylchlorid, Vinylpyridin, Vinylpyrrolidon, Vinylidenchlorid, Vinylidenfluorid, Olefine, Acrylsäure, Acrylsäureester, Acrylamid, Methacrylsäure, Methacrylsäureester, Methacrylamid, Maleinsäure, Maleinsäureester enthalten, und/oder in deren Hauptkette Verknüpfungseinheiten, wie Urethan-, Carbonat-, Ester-, Amid-, Ether-, Thioether-, Acetal-, Keton-oder Sulfongruppierungen wiederkehren.

Leitfähigkeitsmessungen an einem typischen, erfindungsgemäßen Charge-Transfer-Kompex, nämlich an dem 1:2-Komplex (2 Moleküle TCNQ auf 1 Molekül Donor) aus 1,2,4,5-Tetrakis(dimethylamino)benzol und Tetracyanchinodimethan (TCNQ), ein ergaben eine Leitfähigkeit (Einkristall, Vierkontakt-Methode) für diesen Komplex von $\sigma \sim 10^{-1}$ $(\Omega cm)^{-1}$. Die Temperaturabhängigkeit der Leitfähigkeit zeigt, daß es sich um einen typischen Halbleiter handelt.

Beispiel 1

Herstellung von 1,2,4,5-Tetrakis(dimethylamino)benzol

568 mg (2 mmol) 1,2,4,5-Tetraaminobenzol-tetrahydrochlorid wurden in 30 ml Acetonitril unter Schutzgas suspendiert. Die Mischung wurde mit 3 ml einer 36,5 %igen Formaldehyd-Lösung (39,7 mmol) sowie 700 mg (11,1 mmol) Natriumcyanoborhydrid versetzt. Nach 15 Minuten wurde mit einigen Tropfen Eisessig neutralisiert.

Man ließ 3 Stunden rühren und destillierte anschließend das Lösungsmittel im Wasserstrahlvakuum ab. Der Rückstande wurde in 40 ml wäßriger 2n Kaliumhydroxid-Lösung aufgenommen und mit Ether bis zur Farblosigkeit der organischen Phase extrahiert. Die vereinigten organischen Extrakte wurden mit 30 ml 0,5n KOH-Lösung gewaschen. Danach wurden sie dreimal mit je 20 ml 1n Salzsäure ausgeschüttelt. Zur sauren Phase setzte man dann solange festes KOH zu, bis sich die Lösung trübte und alkalische Reaktion zeigte. Dann wurde erneut mit Ether extrahiert, über Kaliumcarbonat getrocknet und das Lösungsmittel abgedampft. Der Rückstand wurde zweimal bei 4 bis 5,3 mbar und 90 bis 100°C Ölbadtemperatur sublimiert: 135 mg (27 % d.Th.), farblose Kristalle vom Schmelzpunkt 95°C.

$^1H$-NMR-Spektrum (360 MHz, $CDCl_3$): $\delta$ = 6,56 (s, 2H, Ar-H),

$\delta$ = 2,76 (s, 24H, N-$CH_3$)
Massenspektrum ($T_Q$ = 90 bis 100°C): m/z (I %) 250 (100; $M^+$), 235 (6), 220 (12), 125 (3; $M^{++}$)
IR-Spektrum (KBr): 2980, 2940, 2820, 2790, 1510 $cm^{-1}$
UV-Spektrum ($CH_3CN$): $\lambda_{max}$ (log $\epsilon$) 242 (4,32), 278 (3,85), 320 (3,68) nm

Beispiel 2

Herstellung des 1,2,4,5-Tetrakis(dimethylamino)benzoldikation-iodid-triiodids

25 mg (0,1 mmol) 1,2,4,5-Tetrakis(dimethylamino)benzol wurden in wenig Acetonitril gelöst und mit einer Lösung von 50,8 mg (0,2 mmol) Iod in Acetonitril versetzt. Aus der dunkelgrünen Lösung schieden sich schwarzviolette Kristalle mit metallischem Glanz ab. Sie wurden aus Acetonitril umkristallisiert. Ausbeute: 29,2 mg (38,5 % d.Th.), Fp. 272°C (ab 195°C Iod-Entwicklung). Die Summenformel entspricht der obengenannten Zusammensetzung eines Dikation-Iodid-Trijodid-Komplexes.
$^1$H-NMR-Spektrum (360 MHz), [$D_6$]DMSO): $\delta$ = 5,85 (s, 2H)
$\delta$ = 3,34 (s, 24H, N-$CH_3$)
IR-Spektrum (KBr): 1563, 1405, 1330, 1157 $cm^{-1}$
UV-Spektrum ($CH_3CN$): $\lambda_{max}$ (log $\epsilon$) 290 (4,74), 368 (4,46), 392 sh (4,43), 5,73 (3,38) nm

Beispiel 3

Herstellung eines Komplexes von 1,2,4,5-Tetrakis(dimethylamino)benzol mit Tetracyanchinodimethan (TCNQ)

25 mg (0,1 mmol) 1,2,4,5-Tetrakis(dimethylamino)benzol wurden in Acetonitril gelöst und mit einer Lösung von 41 mg (0,2 mmol) TCNQ in Acetonitril versetzt. Die dunkelgrüne Lösung wurde auf 80°C erhitzt. Beim Abkühlen auf Raumtemperatur bildeten sich violette glänzende Nadeln. Ausbeute: 29,1 mg (44,2 % d.Th.); Fp.: 213°C (Zersetzung). Die Summenformel entspricht dem Donor-Akzeptor 1:2-Komplex.
IR-Spektrum (KBr): 2160 (C≡N), 1555, 1325, 1125, 953, 825, 690 $cm^{-1}$
UV-Spektrum ($CH_3CN$): $\lambda_{max}$ (log $\epsilon$) 392 (5,29), 676 (3,90), 740 (4,39), 836 (4,61) nm

## Ansprüche

1. Tetrakis(dialkylamino)aromaten der allgemeinen Formeln Ia bis Ic

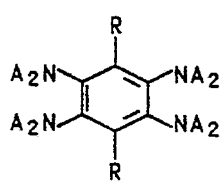

(Ia)

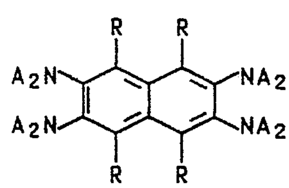

(Ib)

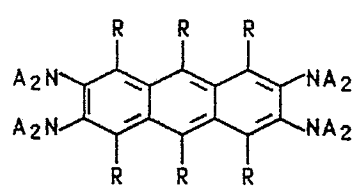

(Ic)

in denen Reste A gleich oder verschieden sein können und für n-$C_1$-$C_6$-Alkyl stehen und die Reste R gleich oder verschieden sein können und für Wasserstoff oder $C_1$-$C_6$-Alkyl stehen, und deren Charge-Transfer-Komplexe mit Elektronen-Akzeptoren.

2. 1,2,4,5-Tetrakis(dialkylamino)benzole der allgemeinen Formel Ia gemäß Anspruch 1, in der A für n-$C_1$-$C_4$-Alkyl steht und R Wasserstoff oder $C_1$-$C_4$-Alkyl bedeutet, und deren Charge-Transfer-Komplexe mit Elektronen-Akzeptoren.

3. 1,2,4,5-Tetrakis(dimethylamino)benzol und dessen Charge-Transfer-Komplexe mit Elektronen-Akzeptoren.

4. Verfahren zur Herstellung der Tetrakis(dialkylamino)aromaten Ia bis Ic und deren Charge-Transfer-Komplexen, dadurch gekennzeichnet, daß man Verbindungen der allgemeinen Formeln IIa bis IIc

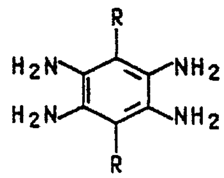

(IIa)

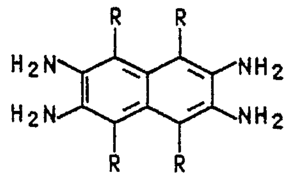

(IIb)

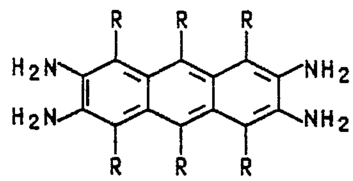

(IIc)

oder deren Säureadditionssalze in einem wasserlöslichen polaren Lösemittel in Gegenwart von Natriumcyanoborhydrid

$NaBH_3CN$

mit einem Aldehyd der allgemeinen Formel III

O
||
A'- C H (III)

in der A' für Wasserstoff oder n-$C_1$-$C_5$-Alkyl steht, reagieren läßt und gegebenenfalls das erhaltene Produkt mit einem Elektronen-Akzeptor unter Bildung eines entsprechenden Charge-Transfer-Komplexes umsetzt.

5. Verfahren nach Anspruch 4 zur Herstellung der Verbindungen Ia bis Ic, in denen A für Methyl steht, dadurch gekennzeichnet, daß man die Verbindungen IIa bis IIc mit wäßrigen Formaldehyd umsetzt.

6. Verfahren nach den Ansprüchen 4 und 5, dadurch gekennzeichnet, daß man in Acetonitril und unter Schutzgas arbeitet.

7. Verfahren nach Anspruch 5, dadurch gekennzeichnet, daß man die Verbindungen IIa bis IIc in Form der Hydrochloridsalze einsetzt und die Reaktion mit einer 30-bis 40 gew.%igen Lösung von Formaldehyd in Wasser durchführt.

8. Verwendung der Charge-Transfer-Komplexe der Tetrakis(dialkylamino)aromaten Ia bis Ic zur Herstellung leitfähiger Polymerer.

9. Charge-Transfer-Komplexe gemäß den Ansprüchen 1 bis 3 aus einem Elektronen-Donator Ia bis Ic einerseits und dem Rest einer Mineralsäure oder einer organischen Säure mit 1 bis 12 C-Atomen andererseits.

10. Charge-Transfer-Komplexe gemäß den Ansprüchen 1 bis 3 aus einem Elektronen-Donator Ia bis Ic einerseits und einem $\pi$-Elektronen-Akzeptor andererseits.